# EUROPEAN PATENT APPLICATION

(11) **EP 4 635 940 A1**
(43) Date of publication of application: **22.10.2025**
(21) Application number: 23903127.1
(22) Date of filing: 27.10.2023
(51) Int. Cl.: C07C 271/04, C07C 239/20, C07C 269/04, C07C 269/06

(54) **PHENOXYCARBAMOYL CHLORIDE COMPOUND AND METHOD FOR PRODUCING SAME**

(30) Priority: 12.12.2022 JP 2022198044
(71) Applicant: Nippon Soda Co., Ltd., Tokyo 100-7010 (JP)
(72) Inventor: NODA, Kaoru, Takaoka-shi, Toyama 933-8507 (JP); TAKEHISA, Katsuma, Takaoka-shi, Toyama 933-8507 (JP); IMAGAWA, Tsutomu, Takaoka-shi, Toyama 933-8507 (JP); MISAKA, Remi, Takaoka-shi, Toyama 933-8507 (JP); ISONO, Marina, Takaoka-shi, Toyama 933-8507 (JP)
(74) Representative: Sonnenhauser, Thomas Martin
(86) International application number: PCT/JP2023/038849
(87) International publication number: WO 2024/127829

(57) **Abstract**

A phenoxycarbamoyl chloride compound useful as an intermediate for the production of a phenoxyurea compound having an insecticidal activity, an acaricidal activity and/or a nematicidal activity is represented by formula (1) (wherein R¹ represents a substituted or unsubstituted C1-6 alkyl group; Y represents a C1-6 haloalkyl group; X represents a halogeno group, a C1-6 alkyl group, or a C1-6 haloalkyl group; n represents an integer of 0 to 4; and X's may be the same as or different from each other when n is 2 or more.)

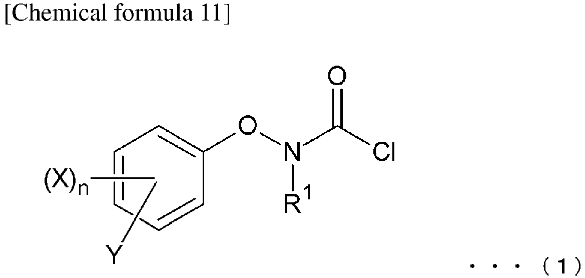

## Description

### TECHNICAL FIELD

The present invention relates to a phenoxycarbamoyl chloride compound, a phenoxyamine compound and a production method of the same. More specifically, the present invention relates to a phenoxycarbamoyl chloride compound which is useful as a production intermediate of a phenoxyurea compound having insecticidal activity, acaricidal activity and/or nematicidal activity, a phenoxyamine compound which is useful as a production intermediate of the phenoxycarbamoyl chloride compound, and an efficient production method of the same in a short process.

Priority is claimed on Japanese Patent Application No. 2022-198044, filed December 12, 2022, the content of which is incorporated herein by reference.

### BACKGROUND ART

Patent Document 1 discloses compounds of the following formula (A) (phenoxyurea compounds), such as 2-{3-[3-bromo-5-(trifluoromethyl)phenoxy]-3-ethylureido}-2-methyl-N-(2,2,2-trifluoroethyl)propanamide, as compounds having insecticidal, acaricidal, or nematicidal activity.

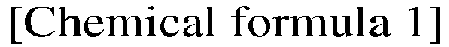

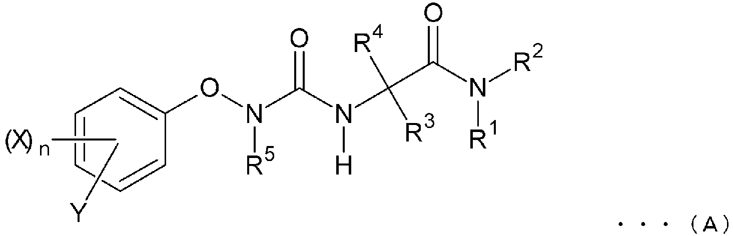

In formula (A), R¹ is a hydrogen atom, a substituted or unsubstituted C1-6 alkyl group, or the like, R² is a hydrogen atom or a C1-6 alkyl group, R³ and R⁴ are each independently a hydrogen atom or a C1-6alkyl group, R⁵ is a substituted or unsubstituted C1-6 alkyl group or the like, Y is a C1-6 haloalkyl group, X is a halogeno group, a C1-6 alkyl group or the like, n is an integer of 0 to 4, and when n is two or more, X may be identical to or different from each other.

Patent Document 1 discloses a compound of the following formula (B1) as a production intermediate of a compound of the following formula (A1).

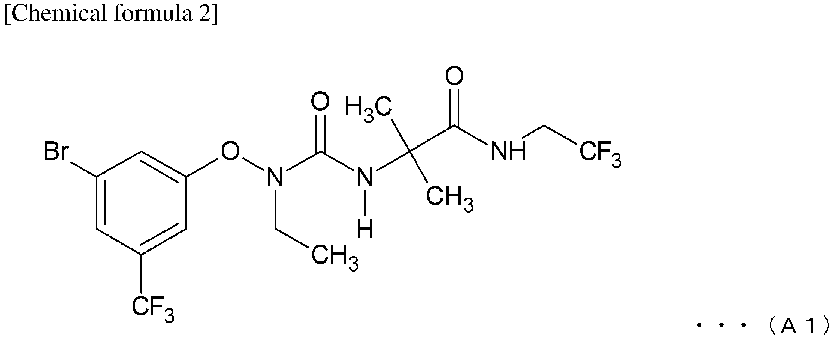

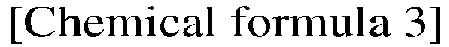

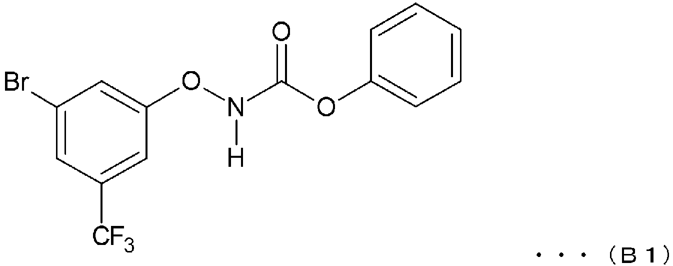

When the compound of formula (B1) is synthesized using a compound of the following formula (C1) as a starting material, appropriate solvents used in each reaction step are different, and therefore solvent replacement is required to be carried out before carrying out each reaction step.

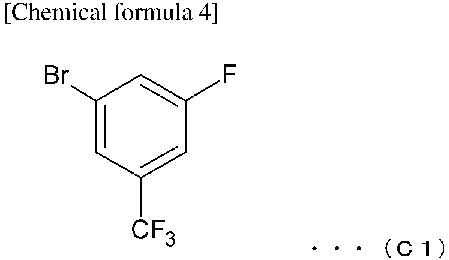

### Citation List

### Patent Document

Patent Document 1: WO 2019/198592 A.

### SUMMARY OF INVENTION

### Technical Problem

The problem of the present invention is to provide a phenoxycarbamoyl chloride compound which is useful as a production intermediate of a phenoxyurea compound having insecticidal activity, acaricidal activity and/or nematicidal activity, a phenoxyamine compound which is useful as a production intermediate of the phenoxycarbamoyl chloride compound, and an efficient production method of the same in a short process.

### Solution to Problem

As a result of extensive research to solve the above-mentioned problems, the present invention encompassing the following aspects has been completed.
(1) A compound of formula (1). 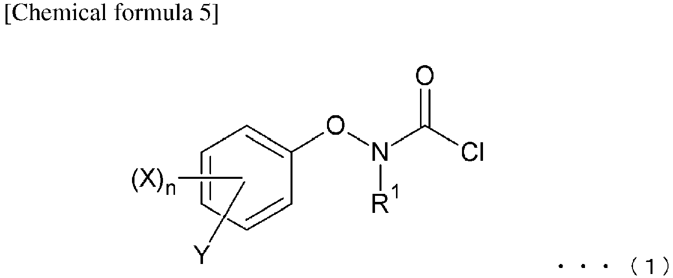 (In formula (1),
   R¹ is a substituted or unsubstituted C1-6 alkyl group,
   Y is a C1-6 haloalkyl group,
   X is a halogeno group, a C1-6 alkyl group, or a C1-6 haloalkyl group,
   n indicates the number of X and is an integer of 0 to 4, and when n is two or more, X may be identical to or different from each other.)
(2) A production method of the compound of formula (1), including:
   chemically reacting a compound of formula (8) with a compound of formula (2) in the presence of a base to obtain an N-alkylated compound;
   hydrolyzing the N-alkylated compound to obtain a compound of formula (3); and then
   chemically reacting the compound of formula (3) with a phosgene or a phosgene equivalent. 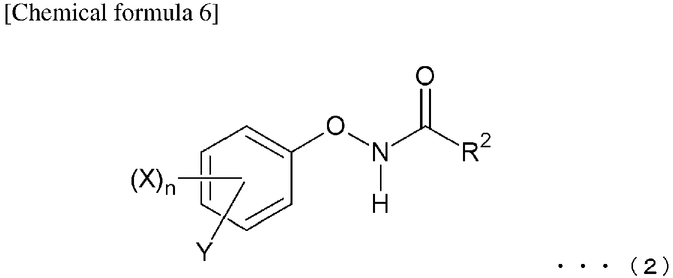 (In formula (2), R² is a C1-8 alkoxy group,
      Y is a C1-6 haloalkyl group,
      X is a halogeno group, a C1-6 alkyl group, or a C1-6 haloalkyl group, and
      n indicates the number of X and is an integer of 0 to 4, and when n is two or more, X may be identical to or different from each other.)

         R¹-Z^{a} (8)
      (In formula (8), R¹ is a substituted or unsubstituted C1-6 alkyl group, and Z^{a} is a halogeno group, or a group of the formula: R¹O-SO₃-.) 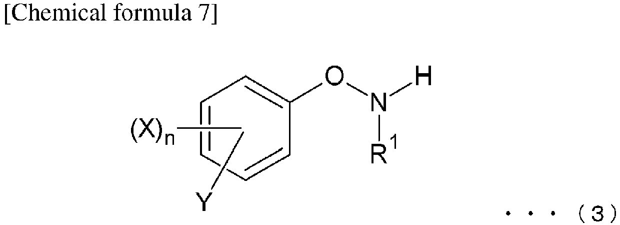 (In formula (3),
         R¹ is a substituted or unsubstituted C1-6 alkyl group, and
         Y, X and n are defined in formula (2).) 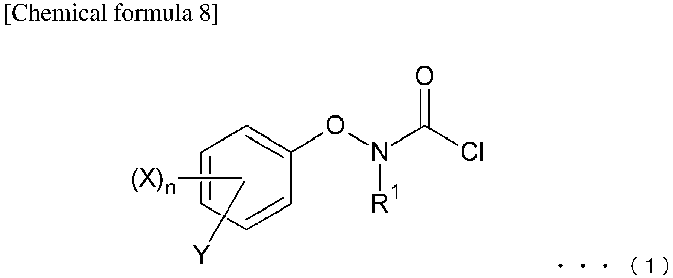 (In formula (1),
            R¹, Y, X and n are defined in formula (3).)
(3) The production method according to (2), further including chemically reacting a compound of formula (4) with a compound of formula (5) in the presence of a base to obtain the compound of formula (2). 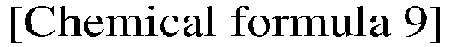 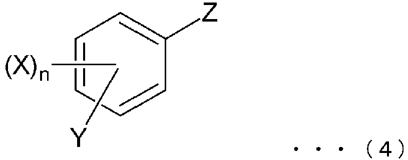 (In formula (4),
   Z is a halogeno group, and
   X, Y, and n are defined in formula (2).) 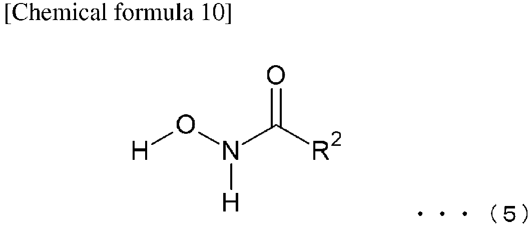 (In formula (5),
      R² is defined in formula (2).)
(4) The production method according to (3), wherein Z is a bromo group.
(5) A compound of formula (3). 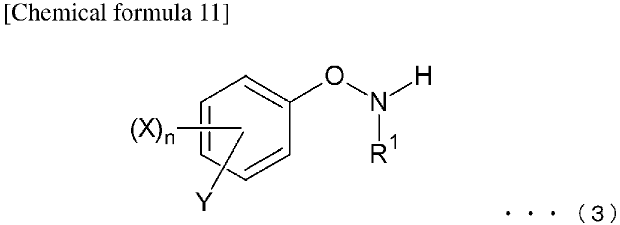 (In formula (3),
   R¹ is a substituted or unsubstituted C1-6 alkyl group,
   Y is a C1-6 haloalkyl group,
   X is a halogeno group, a C1-6 alkyl group, or a C1-6 haloalkyl group, and
   n indicates the number of X and is an integer of 0 to 4, and when n is two or more, X may be identical to or different from each other.)
(6) The compound according to (5), wherein R¹ is a substituted or unsubstituted C2-6 alkyl group.
(7) A production method of the compound of formula (3), including:
   chemically reacting a compound of formula (8) with a compound of formula (2) in the presence of a base to obtain an N-alkylated compound; and
   hydrolyzing the N-alkylated compound. 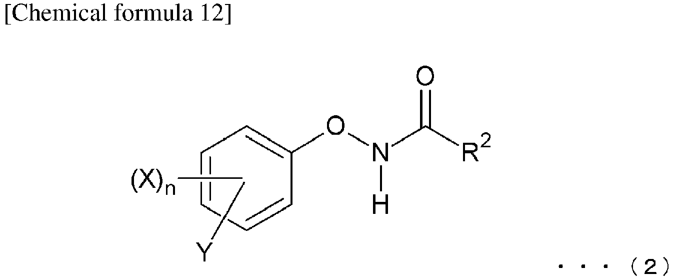 (In formula (2), R² is a C1-8 alkoxy group,
      Y is a C1-6 haloalkyl group,
      X is a halogeno group, a C1-6 alkyl group, or a C1-6 haloalkyl group, and
      n indicates the number of X and is an integer of 0 to 4, and when n is two or more, X may be identical to or different from each other.)

         R¹-Z^{a} (8)
      (In formula (8), R¹ is a substituted or unsubstituted C1-6 alkyl group, and Z^{a} is a halogeno group or a group of the formula: R¹O-SO₃-). 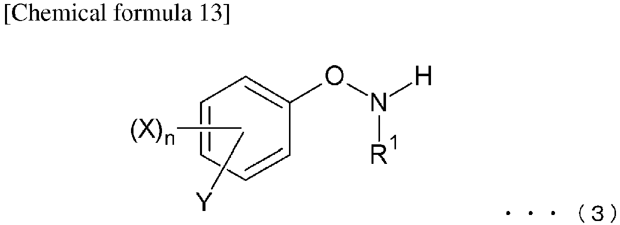 (In formula (3),
         R¹ is a substituted or unsubstituted C1-6 alkyl group, and
         Y, X, and n are defined in formula (2).)
(8) The production method according to (7), wherein R¹ is a substituted or unsubstituted C2-6 alkyl group.
(9) A production method of the compound of formula (1), including chemically reacting a compound of formula (3) with a phosgene or a phosgene equivalent. 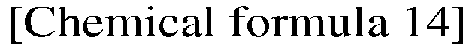 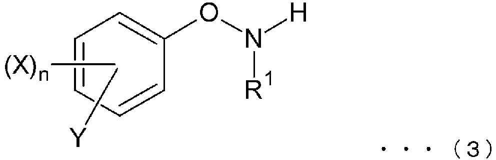 (In formula (3),
   R¹ is a substituted or unsubstituted C1-6 alkyl group,
   Y is a C1-6 haloalkyl group, and
   X is a halogeno group, a C1-6 alkyl group, or a C1-6 haloalkyl group,
   n indicates the number of X and is an integer of 0 to 4, and when n is two or more, X may be identical to or different from each other.) 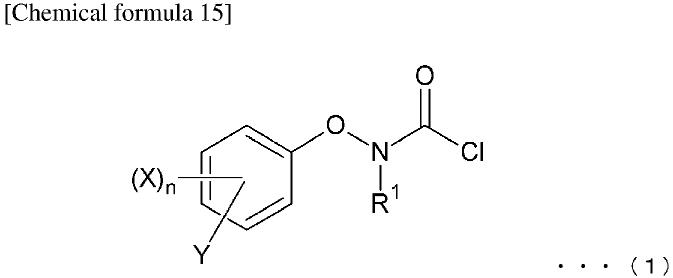 (In formula (1),
      R¹, Y, X and n are defined in formula (3).)

### Advantageous Effects of Invention

The phenoxycarbamoyl chloride compound or the phenoxyamine compound according to the present invention makes it possible to reduce the frequency of solvent replacement to carry out the chemical reaction at each stage until the phenoxyurea compound having insecticidal activity, acaricidal activity and/or nematicidal activity, such as a compound of formula (A1), is obtained.

The production method according to the present invention makes it possible to efficiently obtain a phenoxycarbamoyl chloride compound or a phenoxyamine compound of the present invention in a short process.

### DESCRIPTION OF EMBODIMENTS

A phenoxycarbamoyl chloride compound according to the present invention is a compound of the following formula (1) (hereinafter, may be indicated as compound (1)).

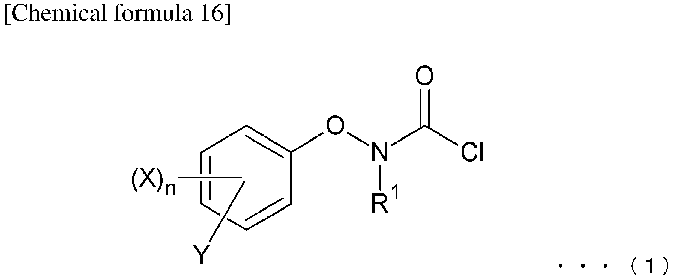

A phenoxyamine compound according to the present invention is a compound of the following formula (3) (hereinafter, may be indicated as compound (3)).

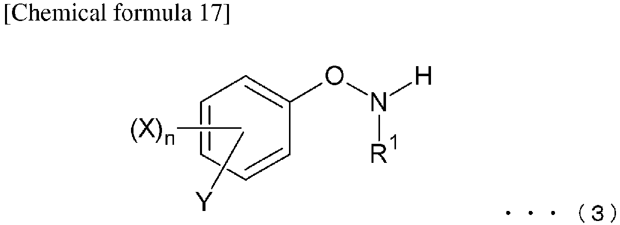

In the formulae, R¹ is a substituted or unsubstituted C1-6 alkyl group, Y is a C1-6 haloalkyl group, X is a halogeno group, a C1-6 alkyl group, or a C1-6 haloalkyl group, and n indicates the number of X and is an integer of 0 to 4, and when n is two or more, X may be identical to or different from each other.

In the present invention, the term "unsubstituted" refers to a group consisting of a mother nucleus. In the case where only the name of a group serving as a mother nucleus is provided without accompanying the term "substituted", this refers to "unsubstituted" unless specifically indicated otherwise.

On the other hand, the term "substituted" means that any hydrogen atom of a group serving as a mother nucleus is substituted with a group (substituent) having a structure that is identical to or different from the mother nucleus. Thus, a "substituent" is another group bound to a group serving as the mother nucleus. The number of substituents may be one or two or more. Two or more substituents may be identical to or different from each other.

The term "C1-6", for example, indicates that the number of carbon atoms of the group serving as the mother nucleus is any of 1 to 6. The number of carbon atoms does not include the number of carbon atoms present in substituents. For example, a butyl group having an ethoxy group as a substituent thereof is classified as a C2 alkoxy C4 alkyl group.

There are no particular limitations on "substituents" provided that they are chemically available and achieve the effects of the present invention.

Specific examples of groups that can be "substituents" include the following groups:
C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group;
C2-6 alkenyl groups such as a vinyl group, a 1-propenyl group, a 2-propenyl group (an allyl group), a 1-butenyl group, a 2-butenyl group, a 3-butenyl group, a 1-methyl-2-propenyl group, and a 2-methyl-2-propenyl group;
C2-6 alkynyl groups such as an ethynyl group, a 1-propynyl group, a 2-propynyl group, a 1-butynyl group, a 2-butynyl group, a 3-butynyl group, and a 1-methyl-2-propynyl group;
C3-6 cycloalkyl groups such as a cyclopropyl group, a cyclobutyl group, a cyclopentyl group, and a cyclohexyl group;
a phenyl group, a naphthyl group;
phenyl C1-6 alkyl groups such as a benzyl group and a phenethyl group;
3- to 6-membered heterocyclyl groups;
3- to 6-membered heterocyclyl C1-6 alkyl groups;
a hydroxy group;
C1-6 alkoxy groups such as a methoxy group, an ethoxy group, a n-propoxy group, an i-propoxy group, a n-butoxy group, a s-butoxy group, an i-butoxy group, and a t-butoxy group;
C2-6 alkenyloxy groups such as a vinyloxy group, an allyloxy group, a propenyloxy group, and a butenyloxy group;
C2-6 alkynyloxy groups such as an ethynyloxy group, and a propargyloxy group;
a phenoxy group, a naphthoxy group;
phenyl C1-6 alkoxy groups such as a benzyloxy group, and a phenethyloxy group;
5- or 6-membered heteroaryloxy groups such as a thiazolyloxy group, and a pyridyloxy group;
5- or 6-membered heteroaryl C1-6 alkyloxy groups such as a thiazolylmethyloxy group, and a pyridylmethyloxy group;
a formyl group;
C1-6 alkylcarbonyl groups such as an acetyl group, and a propionyl group;
a formyloxy group;
C1-6 alkylcarbonyloxy groups such as an acetyloxy group, and a propionyloxy group;
a benzoyl group;
C1-6 alkoxycarbonyl groups such as a methoxycarbonyl group, an ethoxycarbonyl group, a n-propoxycarbonyl group, an i-propoxycarbonyl group, a n-butoxycarbonyl group, and a t-butoxycarbonyl group;
C1-6 alkoxycarbonyloxy groups such as a methoxycarbonyloxy group, an ethoxycarbonyloxy group, a n-propoxycarbonyloxy group, an i-propoxycarbonyloxy group, a n-butoxycarbonyloxy group, and a t-butoxycarbonyloxy group;
a carboxyl group;
halogeno groups such as a fluoro group, a chloro group, a bromo group, and an iodo group;
C1-6 haloalkyl groups such as a chloromethyl group, a chloroethyl group, a trifluoromethyl group, a 1,2-dichloro-n-propyl group, a 1-fluoro-n-butyl group, and a perfluoro-n-pentyl group;
C2-6 haloalkenyl groups such as a 2-chloro-1-propenyl group, and a 2-fluoro-1-butenyl group;
C2-6 haloalkynyl groups such as a 4,4-dichloro-1-butynyl group, a 4-fluoro-1-pentynyl group, and a 5-bromo-2-pentynyl group;
C1-6 haloalkoxy groups such as a trifluoromethoxy group, a 2-chloro-n-propoxy group, and a 2,3-dichlorobutoxy group;
C2-6 haloalkenyloxy groups such as a 2-chloropropenyloxy group, and a 3-bromobutenyloxy group;
C1-6 haloalkylcarbonyl groups such as a chloroacetyl group, a trifluoroacetyl group, and a trichloroacetyl group;
an amino group;
C1-6 alkyl-substituted amino groups such as a methylamino group, a dimethylamino group, and a diethylamino group;
an anilino group, a naphthylamino group;
phenyl C1-6 alkylamino groups such as a benzylamino group, and a phenethylamino group;
a formylamino group;
C1-6 alkylcarbonylamino groups such as an acetylamino group, a propanoylamino group, a butyrylamino group, and an i-propylcarbonylamino group;
C1-6 alkoxycarbonylamino groups such as a methoxycarbonylamino group, an ethoxycarbonylamino group, a n-propoxycarbonylamino group, and an i-propoxycarbonylamino group;
unsubstituted or substituted aminocarbonyl groups such as an aminocarbonyl group, a dimethylaminocarbonyl group, a phenylaminocarbonyl group, and a N-phenyl-N-methylaminocarbonyl group;
imino C1-6 alkyl groups such as an iminomethyl group, a (1-imino)ethyl group, and a (1-imino)-n-propyl group;
substituted or unsubstituted N-hydroxyimino C1-6 alkyl groups such as a N-hydroxy-iminomethyl group, a (1-(N-hydroxy)-imino)ethyl group, a (1-(N-hydroxy)-imino)propyl group, a N-methoxy-iminomethyl group, and a (1-(N-methoxy)-imino)ethyl group;
an aminocarbonyloxy group;
C1-6 alkyl-substituted aminocarbonyloxy groups such as an ethylaminocarbonyloxy group, and a dimethylaminocarbonyloxy group;
a mercapto group;
C1-6 alkylthio groups such as a methylthio group, an ethylthio group, a n-propylthio group, an i-propylthio group, a n-butylthio group, an i-butylthio group, a s-butylthio group, and a t-butylthio group;
C1-6 haloalkylthio groups such as a trifluoromethylthio group, and a 2,2,2-trifluoroethylthio group;
a phenylthio group, a naphthylthio group;
5- or 6-membered heteroarylthio groups such as a thiazolylthio group, and a pyridylthio group;
C1-6 alkylsulfinyl groups such as a methylsulfinyl group, an ethylsulfinyl group, and a t-butylsulfinyl group;
C1-6 haloalkylsulfinyl groups such as a trifluoromethylsulfinyl group, and a 2,2,2-trifluoroethylsulfinyl group;
a phenylsulfinyl group;
5- or 6-membered heteroarylsulfinyl groups such as a thiazolylsulfinyl group, and a pyridylsulfinyl group;
C1-6 alkylsulfonyl groups such as a methylsulfonyl group, an ethylsulfonyl group, and a t-butylsulfonyl group;
C1-6 haloalkylsulfonyl groups such as a trifluoromethylsulfonyl group, and a 2,2,2-trifluoroethylsulfonyl group;
a phenylsulfonyl group;
5- or 6-membered heteroarylsulfonyl groups such as a thiazolylsulfonyl group, and a pyridylsulfonyl group;
C1-6 alkylsulfonyloxy groups such as a methylsulfonyloxy group, an ethylsulfonyloxy group, and a t-butylsulfonyloxy group;
C1-6 haloalkylsulfonyloxy groups such as a trifluoromethylsulfonyloxy group, and a 2,2,2-trifluoroethylsulfonyloxy group;
tri C1-6 alkyl-substituted silyl groups such as a trimethylsilyl group, a triethylsilyl group, and a t-butyldimethylsilyl group;
a triphenylsilyl group;
a cyano group; and a nitro group.

Any hydrogen atom of the "substituent" may be substituted with a group having a different structure. Examples of such a substituent include C1-6 alkyl groups, C1-6 haloalkyl groups, C1-6 alkoxy groups, C1-6 haloalkoxy groups, halogeno groups, a cyano group, and a nitro group.

The "3- to 6-membered heterocyclyl group" contains, as a ring member atom, one to four hetero atoms selected from the group consisting of a nitrogen atom, an oxygen atom and a sulfur atom. The heterocyclyl group may be monocyclic or polycyclic. If at least one ring of a polycyclic heterocyclyl group is a hetero ring, the remaining rings thereof may be any of saturated alicyclic rings, unsaturated alicyclic rings and aromatic rings. Examples of the "3- to 6-membered heterocyclyl group" include 3- to 6-membered saturated heterocyclyl groups, 5- or 6-membered heteroaryl groups, and 5- or 6-membered partially unsaturated heterocyclyl groups.

Examples of the "3- to 6-membered saturated heterocyclyl groups" include an aziridinyl group, an epoxy group, a pyrrolidinyl group, a tetrahydrofuranyl group, a thiazolidinyl group, a piperidyl group, a piperazinyl group, a morpholinyl group, a dioxolanyl group, and a dioxanyl group.

Examples of the "5-membered heteroaryl groups" include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the "6-membered heteroaryl groups" include a pyridyl group, a pyrazinyl group, a pyrimidinyl group, a pyridazinyl group, and a triazinyl group.

Examples of the "5- to 6-membered unsaturated heterocyclyl groups" include: 5-membered unsaturated heterocyclyl groups such as a pyrrolinyl group, a dihydrofuranyl group, a dihydrothiophenyl group, an imidazolynyl group, a pyrazolynyl group, an oxazolynyl group, isooxazolynyl group, a thiazolynyl group, and an isothiazolynyl group; and 6-membered unsaturated heterocyclyl groups such as a dihydropyranyl group.

In formula (1), R¹ is a substituted or unsubstituted C1-6 alkyl group.

The "C1-6 alkyl group" as R¹ may be a linear chain or a branched chain. Examples of the "C1-6 alkyl group" as R¹ include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, and an i-hexyl group.

Preferable examples of a substituent on the "C1 -6 alkyl group" as R¹ include halogeno groups, a hydroxy group, C1-6 alkoxy groups, C1-6 haloalkoxy groups, C3-6 cycloalkyl groups, a phenyl group, 5-membered heteroaryl groups, 6-membered heteroaryl groups, a cyano group; a phenyl group substituted with at least one substituent selected from C1-6 alkyl groups, C1-6 alkoxy groups, halogeno groups, C1-6 haloalkyl groups, and C1-6 haloalkoxy groups; 5-membered heteroaryl groups substituted with at least one substituent selected from C1-6 alkyl groups, C1-6 alkoxy groups, halogeno groups, C1-6 haloalkyl groups, and C1-6 haloalkoxy groups; and 6-membered heteroaryl groups substituted with at least one substituent selected from C1-6 alkyl groups, C1-6 alkoxy groups, halogeno groups, C1-6 haloalkyl groups, and C1-6 haloalkoxy groups.

In formula (1), Y is a C1-6 haloalkyl group (C1-6 alkyl group substituted with at least one halogeno group).

Examples of the "C1-6 haloalkyl group" as Y include a chloromethyl group, a chloroethyl group, a trifluoromethyl group, a 1,2-dichloro-n-propyl group, a 1-fluoro-n-butyl group, and a perfluoro-n-pentyl group, and a trifluoromethyl group is preferable.

In formula (1), X is a halogeno group, a C1-6 alkyl group, or a C1-6 haloalkyl group, and is preferably a halogeno group.

Examples of the "halogeno group" as X include a fluoro group, a chloro group, a bromo group, and an iodo group, and a bromo group is preferable. In formula (1), n indicates the number of X and is an integer of 0 to 4, and preferably 1. When n is two or more, X may be identical to or different from each other.

Examples of the "C1-6 alkyl group" as X include the same as those mentioned as R¹.

Examples of the "C1-6 haloalkyl group" as X include the same as those mentioned as Y.

Although the compound (1) is not limited by the production method, the compound (1) can be obtained efficiently dependent on the production method according to the present invention.

The production method according to the present invention includes: chemically reacting a compound of the following formula (2) (hereinafter, may be indicated as compound (2)) with a compound of the following formula (8) (hereinafter, may be indicated as compound (8)) in the presence of a base to obtain an N-alkylated compound; hydrolyzing the N-alkylated compound to obtain a compound of the following formula (3) (hereinafter, may be indicated as compound (3)); and then chemically reacting the compound of formula (3) with a phosgene or a phosgene equivalent.

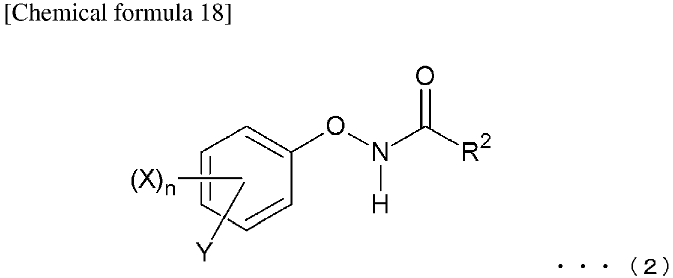

(In formula (2), R² is a C1-8 alkoxy group.

Examples of the "C1-8 alkoxy group" as R² include a methoxy group, an ethoxy group, a propoxy group, and a butoxy group. Among them, R² is preferably a C1-3 alkoxy group.

Y, X and n in formula (2) are the same as Y, X and n in formula (1).

In formula (2), X is preferably a halogeno group, and more preferably a bromo group.

In formula (2), n is preferably 1.

In formula (2), Y is preferably a halomethyl group, and more preferably a trifluoromethyl group.

R¹-Z^{a} (8)

In formula (8), R¹ is a substituted or unsubstituted C1-6 alkyl group, and Z^{a} is a halogeno group or a group of the formula: R¹O-SO₃-.

Specific examples of the compound (8) include: alkyl halides such as methyl bromide, methyl chloride, ethyl bromide, and ethyl chloride; and dialkyl sulfates such as dimethyl sulfate and diethyl sulfate. Among these, the compound (8) is preferably a di-C1-2 alkyl sulfate, and more preferably diethyl sulfate.

Examples of the base used when the compound (2) is chemically reacted with the compound (8) include: inorganic bases such as metal hydroxides (such as KOH, NaOH, and Ca(OH)₂), metal hydrides (such as LiH and NaH), metal carbonates (such as Na₂CO₃ and K₂CO₃), and metal hydrogen carbonates (such as NaHCO₃ and K₂CO₃); and organic bases such as metal alkoxides (such as NaOMe, and t-BuOK), metal amides (such as NaNH₂ and LDA), alkyl metal compounds (such as n-BuLi and Me₃Al), alkylamines (such as Et₃N, TMEDA, piperidine, and DABCO), and heterocyclic amines (such as DBU, pyridine, imidazole, and DMAP). Among these, metal hydroxides are preferably used, and potassium hydroxide is more preferably used.

The base used when the compound (2) is chemically reacted with the compound (8) may be added dropwise in the form of an aqueous solution to a mixed solution containing the compound (2) and the compound (8) under stirring.

Alternatively, the compound (8) may be added dropwise to a mixed solution containing the compound (2) and the base.

A solvent may be used when the compound (2) is chemically reacted with the compound (8). Examples of the solvent include: non-polar solvents such as hexane, benzene, toluene, chloroform, tetrachloromethane, and diethyl ether; protic polar solvents such as water, methanol, and ethanol; and aprotic polar solvents such as dimethyl sulfoxide, dimethylformamide, and hexamethylphosphoric triamide. Among these, non-polar solvents and/or aprotic polar solvents are preferably used, and toluene and/or dimethyl sulfoxide are more preferably used.

When the compound (2) is chemically reacted with the compound (8), a quaternary ammonium salt may be present. There is a case in which the presence of a quaternary ammonium salt accelerates the chemical reaction. Examples of the quaternary ammonium salt include benzyltributylammonium chloride, benzyltrimethylammonium chloride, benzyltriethylammonium chloride, tetramethylammonium chloride, tetrapropylammonium hydroxide, tetrabutylammonium bromide, and trimethylphenylammonium chloride, and benzyltributylammonium chloride is preferably used.

When the compound (2) is chemically reacted with the compound (8), the temperature is preferably 0°C to 150°C, more preferably 5 to 60°C, and even more preferably 10 to 40°C, and the pressure is not particularly limited, and may be, for example, ordinary pressure, and the reaction time is approximately one hour to eight hours, and the reaction is preferably carried out under stirring.

The process of chemically reacting the compound (2) with the compound (8) preferably includes: a process a in which the compound (8) is added to a mixture solution containing the compound (2) and a solvent while carrying out stirring; a process b in which a quaternary ammonium salt is further added to the mixture solution obtained in the process a; and a process c in which an aqueous solution of a base is further added dropwise to the mixture solution obtained in the process b while carrying out stirring.

Alternatively, the process of chemically reacting the compound (2) with the compound (8) preferably includes any of a process d in which the compound (8) is added dropwise to a mixture solution containing the compound (2), a solvent, a base, and water while carrying out stirring; a process e in which the compound (8) and an aqueous solution of a base are simultaneously added dropwise to a reaction solution obtained in the process d; and a process f in which an aqueous solution of a base is added dropwise to the reaction solution obtained in the process d. The dropwise-addition process is preferably carried out over approximately five minutes to three hours.

After the process of chemically reacting the compound (2) with the compound (8), an extraction process, a concentration process, and a purification process by column chromatography or the like may be carried out as necessary.

The amount of the compound (8) relative to one mol of the compound (2) is preferably 80% by mol to 320% by mol, and more preferably 150% by mol to 250% by mol.

The amount of the base relative to one mol of the compound (2) is preferably 80% by mol to 500% by mol, and more preferably 200% by mol to 400% by mol.

The N-alkylated compound obtained by chemically reacting the compound (2) with the compound (8) is a compound of formula (3p) (hereinafter, may be indicated as compound (3p)).

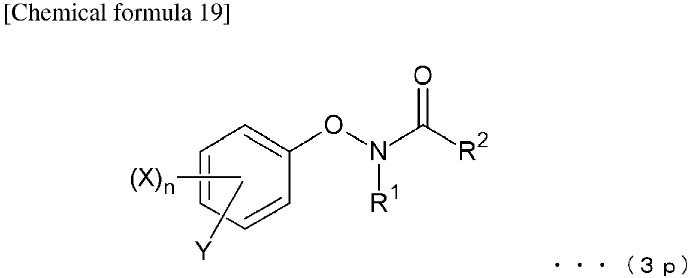

In formula (3p), R¹ is a substituted or unsubstituted C1-6 alkyl group, R² is a C1-8 alkoxy group, and Y, X and n are the same as Y, X and n in formula (1).

In formula (3p), R¹ is preferably an ethyl group.

In formula (3p), R² is preferably a methoxy group.

In formula (3p), X is preferably a halogeno group, and more preferably a bromo group.

In formula (3p), n is preferably 1.

In formula (3p), Y is preferably a halomethyl group, and more preferably a trifluoromethyl group.

When the compound (3p) is subjected to a hydrolysis reaction, a solvent may be used. Examples of the solvent include non-polar solvents such as hexane, benzene, toluene, chloroform, tetrachloromethane, and diethyl ether; protic polar solvents such as water, methanol, and ethanol; and aprotic polar solvents such as dimethyl sulfoxide, dimethylformamide, and hexamethylphosphoric triamide. Among these, non-polar solvents are preferably used, and toluene is more preferably used.

There is a case in which the presence of a base promotes the hydrolysis reaction of compound (3p). Examples of the base include the same as those already mentioned when the compound (2) is chemically reacted with the compound (8).

A quaternary ammonium salt may be present during the hydrolysis reaction of the compound (3p). There is a case in which the presence of the quaternary ammonium salt accelerates the hydrolysis reaction. Examples of the quaternary ammonium salt include the same as those already mentioned when the compound (2) is chemically reacted with the compound (8).

When the compound (3p) is subjected to a hydrolysis reaction, the temperature is preferably 10°C to 100°C, and more preferably 40°C to 80°C, the pressure is not particularly limited and may be, for example, ordinary pressure, the reaction time is preferably approximately one hour to 20 hours, and the hydrolysis reaction is preferably carried out under stirring.

The process of subjecting the compound (3p) to a hydrolysis reaction includes a process in which an aqueous solution of a base and an aqueous solution of a quaternary ammonium salt are added to a mixture solution containing the compound (3p) and a solvent, followed by heating and stirring the mixture solution.

Alternatively, the process of subjecting the compound (3p) to a hydrolysis reaction includes a process in which water and a solvent are added to a reaction solution obtained by the process of chemically reacting the compound (2) with the compound (8), followed by subjecting the resultant to an extraction.

After the process of subjecting the compound (3p) to a hydrolysis reaction, an extraction process, a concentration process, and a purification process by column chromatography may be carried out.

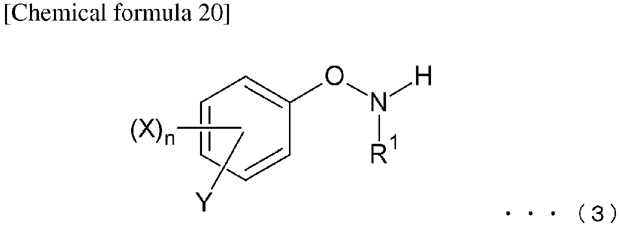

In formula (3), R¹ is a substituted or unsubstituted C1-6 alkyl group, and Y, X and n are the same as Y, X and n in formula (1). In formula (3), R¹ is preferably a substituted or unsubstituted C2-6 alkyl group

In formula (3), R¹ is preferably an ethyl group.

In formula (3), X is preferably a halogeno group, and more preferably a bromo group.

In formula (3), n is preferably 1.

In formula (3), Y is preferably a halomethyl group, and more preferably a trifluoromethyl group.

Although the compound (3) can be produced by the above-mentioned method, the compound (3) may be produced by another method using other starting materials.

A phosgene (carbonyl chloride) is a compound of the molecular formula: COCl₂. A phosgene equivalent is a compound that plays almost the same role as phosgene. Examples of the phosgene equivalent include fluorine-containing carbonic acid diesters, diphosgene, triphosgene (bis(trichloromethyl)carbonate), N-substituted imidoyl chlorides, trichloromethyl chloroformate, and 1,1'-carbonyldiimidazole.

When the compound (3) is chemically reacted with a phosgene or a phosgene equivalent, a solvent may be used. Examples of the solvent include non-polar solvents such as hexane, benzene, toluene, chloroform, tetrachloromethane, and diethyl ether, and toluene is preferably used.

When the compound (3) is chemically reacted with the phosgene or the phosgene equivalent, the temperature is preferably 0°C to 100°C, and more preferably 30°C to 70°C, the pressure is not particularly limited and may be, for example, ordinary pressure, the reaction time is, for example, approximately 30 minutes to one hour, and the chemical reaction is carried out under stirring.

The process of chemically reacting the compound (3) with a phosgene or a phosgene equivalent preferably includes: a process in which the phosgene or the phosgene equivalent is blown into a solvent at a temperature of about 5°C to 15°C over 10 minutes to 30 minutes; a process in which a mixture solution containing the compound (3) and a solvent is added dropwise at a temperature of about 40°C to 60°C over five minutes to 30 minutes to the solvent into which the phosgene or the phosgene equivalent has been blown; a process in which stirring is carried out at a temperature of about 50°C to 60°C for about 30 minutes to one hour; and a process in which the obtained reaction solution is purged by blowing nitrogen gas thereinto, for example.

The amount of the phosgene or the phosgene equivalent relative to one mol of the compound (3) is preferably 80% by mol to 320% by mol, and more preferably 100% by mol to 200% by mol.

The compound (1) is useful as a production intermediate of a phenoxyurea compound. For example, a phenoxyurea compound (see the following formula (6)) can be obtained by reacting the compound (1) with an amino group-containing compound such as 2-aminio-2-methyl-N-(2,2,2-trifluoroethyl)propanamide or 2-aminoacetamide.

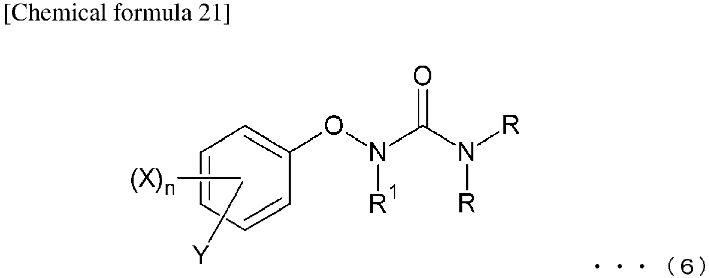

In formula (6), R is each independently a hydrogen atom or an organic group (such as a 2-methyl-1-oxo-1-[(2,2,2-trifluoroethyl)amino]propan-2-yl group, or a 2-amino-2-oxoethyl group).

In formula (6), R¹, Y, X and n are the same as R¹, Y, X and n in formula (1).

When the compound (1) is reacted with an amino group-containing compound, a solvent may be used. Examples of the solvent include non-polar solvents such as hexane, benzene, toluene, chloroform, tetrachloromethane, and diethyl ether.

Although the compound of formula (2) is not limited by the production method thereof, the compound of formula (2) can be obtained efficiently by chemically reacting a compound of the following formula (4) (hereinafter, may be indicated as compound (4)) with a compound of the following formula (5) (hereinafter, may be indicated as compound (5)) in the presence of a base, for example.

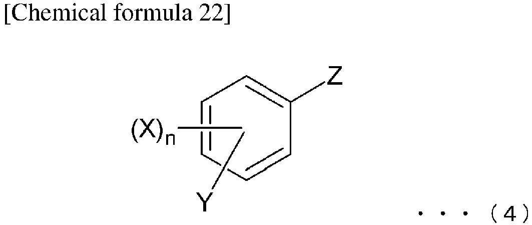

In formula (4), Z is a halogeno group.

Examples of the "halogeno group" as Z include a fluoro group, a chloro group, a bromo group, and an iodo group. Z is preferably a bromo group.

In formula (4), X, Y and n are the same as X, Y and n in formula (2).

In formula (4), X is preferably a halogeno group, and more preferably a bromo group.

In formula (4), n is preferably 1.

In formula (4), Y is preferably a halomethyl group, and more preferably a trifluoromethyl group.

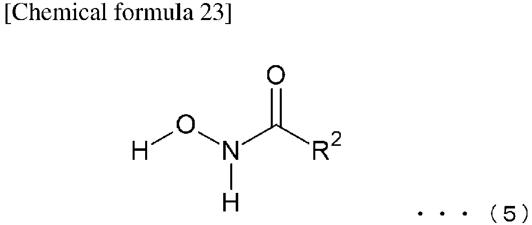

In formula (5), R² is the same as R² in formula (2).

Namely, the compound (5) is a C1-8 alkyl hydroxy carbamate.

The compound (5) of formula (5) is preferably a C1-3 alkyl hydroxy carbamate, and more preferably a methyl hydroxy carbamate or an isopropyl hydroxy carbamate.

Examples of the base used when the compound (4) is chemically reacted with the compound (5) include: inorganic bases such as metal hydroxides (such as KOH, NaOH, and Ca(OH)₂), metal hydrides (such as LiH and NaH), metal carbonates (such as Na₂CO₃ and K₂CO₃), and metal hydrogen carbonates (such as NaHCO₃ and K₂CO₃); and organic bases such as metal alkoxides (such as NaOMe, and t-BuOK), metal amides (such as NaNH₂ and LDA), alkyl metal compounds (such as n-BuLi and Me₃Al), alkylamines (such as Et₃N, TMEDA, piperidine, and DABCO), and heterocyclic amines (such as DBU, pyridine, imidazole, and DMAP). Among these, metal hydroxides are preferably used, and potassium hydroxide is more preferably used.

When the compound (4) is chemically reacted with the compound (5), a solvent may be used. Examples of the solvent include: non-polar solvents such as toluene; aprotic polar solvents such as ethyl acetate, dichloromethane, acetone, dimethyl sulfoxide, acetonitrile, dimethylformamide, tetrahydrofuran, and N-methylpyrrolidone; and protic polar solvents such as water, methanol, and ethanol. Among these, non-polar solvents and/or aprotic polar solvents are preferably used, and toluene and/or dimethyl sulfoxide are more preferably used.

When the compound (4) is chemically reacted with the compound (5), the temperature is preferably 0°C to 120°C, more preferably 40°C to 70°C, and even more preferably 50°C to 65°C, the pressure is not particularly limited and may be, for example, ordinary pressure, the reaction time is preferably about three hours to ten hours, and the chemical reaction is preferably carried out under stirring.

The process of chemically reacting the compound (4) with the compound (5) may include a process in which a mixture solution containing the compound (4), the compound (5), a solvent, and a base is stirred, or a process in which a mixture solution containing the compound (5) and a solvent is added dropwise to a mixture solution containing the compound (4), a solvent, and a base over about three hours to five hours, for example, followed by stirring the resultant.

After the process of chemically reacting the compound (4) with the compound (5), an extraction process, a concentration process, and a purification process by column chromatography or the like may be carried out as necessary.

The amount of the compound (5) relative to one mol of the compound (4) is preferably 80% by mol to 320% by mol, and more preferably 100% by mol to 200% by mol.

### [Examples]

Hereinafter, the present invention is described more specifically with reference to examples. The present invention is not limited to the following examples.

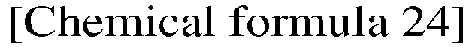

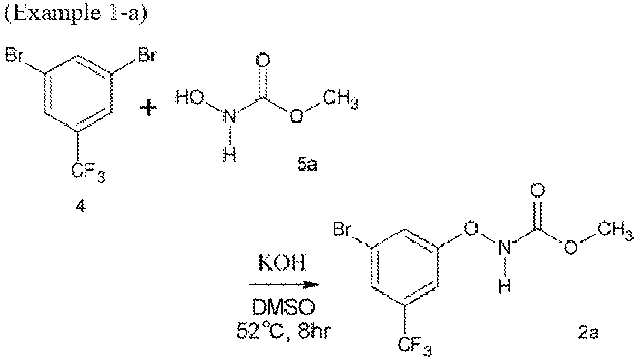

1,3-Dibromo-5-(trifluoromethyl)benzene (30.4 g) (compound 4), dimethyl sulfoxide (300 mL), methyl hydroxy carbamate (18.2g) (compound 5a), and 85% by mass of powdered potassium hydroxide (26.4 g) were mixed and stirred at 52°C for eight hours. Water (300 mL), toluene (300 mL), and 35% by mass of hydrochloric acid (31.2 g) were added to the obtained liquid and stirred. Thereafter, the toluene layer was separated to obtain a toluene solution of compound 2a (at a yield of 95%). The concentrate obtained by distilling off the toluene was purified by silica gel chromatography to obtain compound 2a.
¹H-NMR (CDCl₃/TMS, δ (ppm)) 7.85 (s, 1H), 7.47-7.48 (m, 1H), 7.44-7.46 (m, 1H), 7.30-7.32 (m, 1H), 3.86 (s, 3H)
¹³C-NMR (CDCl₃/TMS, δ (ppm)) 160.5, 157.7, 133.2, 124.2, 123.2, 121.5, 120.0, 109.6, 53.8

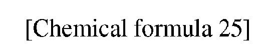

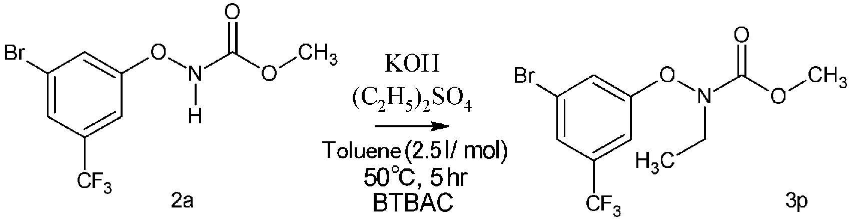

Compound 2a (16.4 g) was dissolved in toluene (50 mL). Diethyl sulfate (8.06 g) was added to the obtained solution under stirring, and then an aqueous solution (0.63 g) containing 50% by mass of benzyltributylammonium chloride was further added thereto. A solution obtained by dissolving granular potassium hydroxide (2.61 g) in water (2.61 g) was added dropwise to the resultant liquid. Then, the resultant was heated and stirred at 50°C for five hours. Water (30 g) was added to the resultant liquid to separate the toluene layer, thereby obtaining a toluene solution of compound 3p (at a yield of 90%).

The toluene solution of compound 3p was concentrated, purified by column chromatography and then subjected to NMR analysis.
¹H-NMR (CDCl₃/TMS, δ (ppm)): 7.43 (s, 1H), 7.39-7.41 (m, 1H), 7.24 (s, 1H), 3.81 (s, 3H), 3.71 (q, 2H), 1.23 (t, 3H)

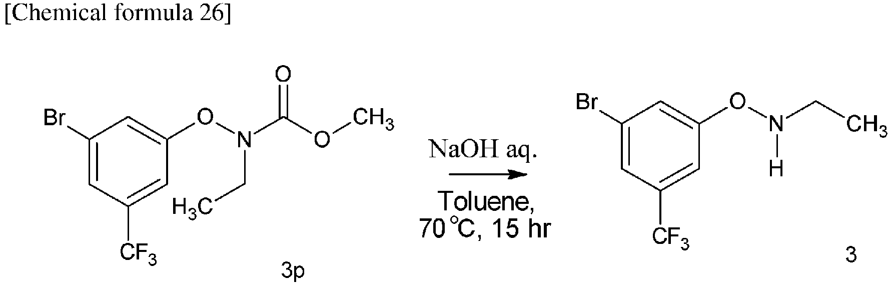

Compound 3p (10.3 g) was dissolved in toluene (50 ml). To the resultant solution, an aqueous solution (60g) containing 10% by mass of sodium hydroxide and an aqueous solution (0.94 g) containing 50% by mass of benzyltributylammonium chloride were added. The resultant mixture was heated and stirred at 70°C for 15 hours. Water (50 g) was added to the resultant liquid to separate the toluene layer, thereby obtaining a toluene solution of compound 3 (at a yield of 91%).

The toluene solution of compound 3 was concentrated, purified by column chromatography and then subjected to NMR analysis.
¹H-NMR (CDCl₃/TMS, δ (ppm)): 7.47-7.50 (m, 1H), 7.29-7.32 (m, 2H), 6.00 (t, 1H), 3.15 (dt, 2H), 1.16 (t, 3H)

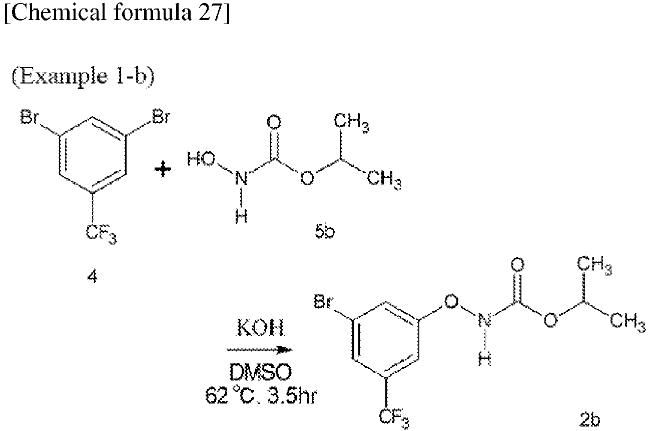

A toluene solution (8.79g) containing 69% by mass of 1,3-dibromo-5-(trifluoromethyl)benzene, dimethyl sulfoxide (10 mL), and 85% by mass of a pelletized potassium hydroxide (5.28 g) were mixed and stirred at 62°C for ten minutes. A solution of isopropyl hydroxy carbamate (4.81 g) in dimethyl sulfoxide (4.0 mL) was added dropwise to the resultant mixture over four hours. The resultant was then stirred at 62°C for 3.5 hours. Next, water (14 mL), toluene (20 mL), and 35% by mass of hydrochloric acid (5.77 g) were added to the resultant and stirred at 45°C. The toluene layer was separated. Water (6 mL) was added to the toluene layer and stirred. The toluene layer was separated. A toluene solution of compound 2b (at a yield of 95%) was obtained. Toluene was distilled off from the resultant, and the residue was purified by silica gel chromatography to obtain compound 2b.
¹H-NMR (CDCl₃/TMS, δ (ppm)): 7.68 (s, 1H), 7.47 (s, 1H), 7.44 (s, 1H), 7.30-7.31 (m, 1H), 5.02-5.12 (spt, 1H), 1.31-1.32 (d, 6H)

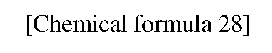

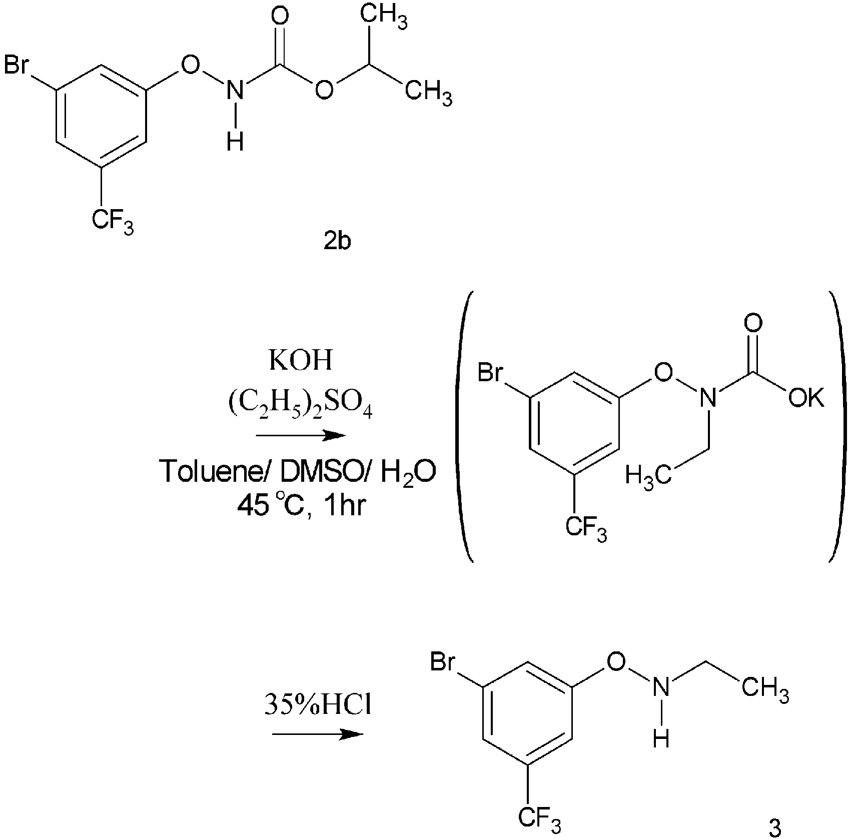

A toluene solution (5.07 g) containing 68% by mass of compound 2b, dimethyl sulfoxide (7.84 g), water (2.02 g), and 85% by mass of a pelletized potassium hydroxide (2.62 g) were placed in a 50 mL four-necked flask. The mixture was heated and diethyl sulfate (1.55 g) was added dropwise over 45 minutes at 40°C. Then, diethyl sulfate (1.38 g) and an aqueous solution (2.19g) containing 50% by mass of potassium hydroxide were added dropwise simultaneously over 30 minutes. Then, the resultant liquid was stirred for one hour. The resultant liquid was cooled, and water (2.02 g) and toluene (6.18 g) were added thereto at 0°C to 5°C, to separate the aqueous layer and the toluene layer. In the toluene layer, 0.7% by mass of compound 3 was contained. Toluene (6.23 g) and 35% by mass of hydrochloric acid (4.17g) were added to the aqueous layer. The pH became 4.1. The toluene layer was separated to obtain a toluene solution of compound 3 (at a yield of 87%).

The toluene solution of compound 3 was concentrated, purified by column chromatography and then subjected to NMR analysis.
¹H-NMR (CDCl₃/TMS, δ (ppm)): 7.47-7.50 (m, 1H), 7.29-7.32 (m, 2H), 6.00 (t, 1H), 3.15 (dt, 2H), 1.16 (t, 3H)

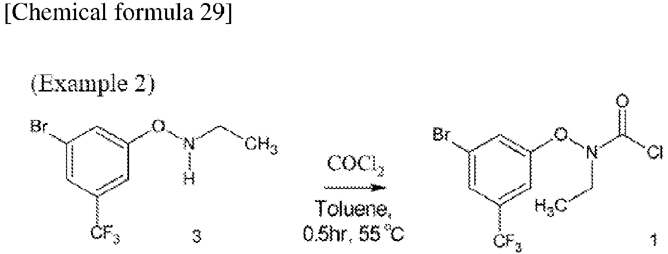

Phosgene (3 g) was blown into toluene (20 mL) at a temperature of 5°C to 10°C over 15 minutes. Toluene (17.5 g) was added to a toluene solution (12g) containing 58% by mass of compound 3, and then added dropwise at 50°C to 54°C over ten minutes. The resultant was then stirred at 54°C to 57°C for 30 minutes. The resultant liquid was purged by blowing nitrogen gas thereinto. Then, the resultant liquid was concentrated to obtain compound 1 (20 g).
¹H-NMR (CDCl₃/TMS, δ (ppm)): 7.50 (s, 1H), 7.40 (s, 1H), 7.23 (s, 1H), 3.85-3.90 (q, 2H), 1.32-1.36 (t, 3H)
¹³C-NMR (CDCl₃/TMS, δ (ppm)): 158.5, 150.9 (C=O, HMBC correlation), 133.2-134.0, 123.9-124.0, 123.8, 121.3, 119.9, 109.5-109.6, 47.24, 11.82
IR (cm⁻¹) : 2983.62, 2110.72, 1743.32 (C=O), 1613.44, 1586.92, 1438.11, 1383.95, 1319.53, 1306.64, 1239.24, 1173.97, 1135.15, 1105.6, 1088.08, 1076.55, 968.26, 931.68, 917.61, 860.57, 806.78, 784.22, 731.36, 713.64, 697.4, 688.99, 660.61, 549.54, 521.62, 506.89, 478.91, 467.14, 443.95, 406.72

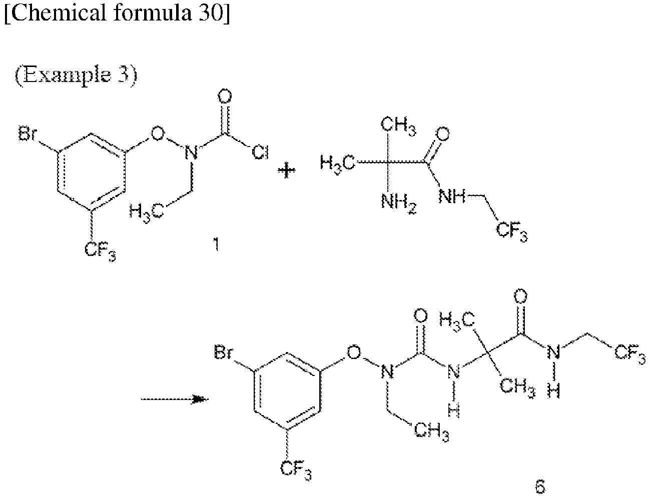

2-Amino-2-methyl-N-(2,2,2-trifluoroethyl)propanamide (23.6 g) was dissolved in toluene (110 mL). The resultant solution was added dropwise to the toluene solution of compound 1. The resultant was then stirred at 50°C to 50°C for six hours. Water (10 mL) was added to the resultant liquid. The toluene layer was separated to obtain a toluene solution of compound 6 (at a yield of 75%).

The toluene solution of compound 6 was concentrated and purified by silica gel chromatography to obtain compound 6 (2-{3-[3-bromo-5-(trifluoromethyl)phenoxy]-3-ethylureido }-2-methyl-N-(2,2,2-trifluoroethyl)propanamide).

¹H-NMR (CDCL₃/TMS, δ (ppm)): 7.51 (s, 2H), 7.33 (s, 1H), 7.12 (t, 1H), 5.99 (s, 1H), 3.94 (dq, 2H), 3.67 (q, 2H), 1.57 (s, 6H), 1.17 (t, 3H).

### INDUSTRIAL APPLICABILITY

The phenoxycarbamoyl chloride compound (compound of formula (1)) according to the present invention is useful as a production intermediate of a phenoxyurea compound having insecticidal activity, acaricidal activity and/or nematicidal activity. The phenoxyamine compound (compound of formula (3)) according to the present invention is useful as a production intermediate of the phenoxycarbamoyl chloride compound (compound of formula (1)).

## Claims

1. A compound of formula (1): 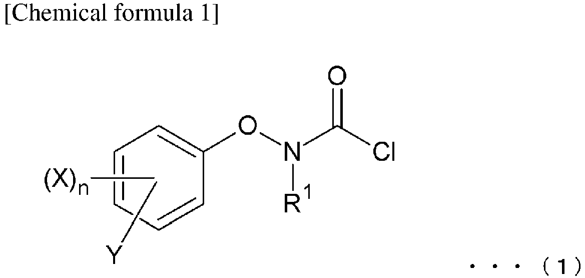 (in formula (1),
R¹ is a substituted or unsubstituted C1-6 alkyl group,
Y is a C1-6 haloalkyl group,
X is a halogeno group, a C1-6 alkyl group, or a C1-6 haloalkyl group,
n indicates a number of X and is an integer of 0 to 4, and when n is two or more, X may be identical to or different from each other.)

2. A production method of a compound of formula (1), comprising:
chemically reacting a compound of formula (8) with a compound of formula (2) in the presence of a base to obtain an N-alkylated compound;
hydrolyzing the N-alkylated compound to obtain a compound of formula (3); and then
chemically reacting the compound of formula (3) with a phosgene or a phosgene equivalent, 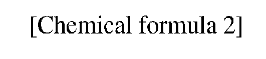 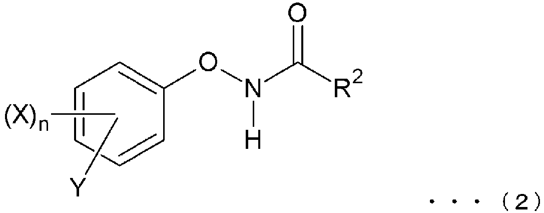 (in formula (2), R² is a C1-8 alkoxy group,
Y is a C1-6 haloalkyl group,
X is a halogeno group, a C1-6 alkyl group, or a C1-6 haloalkyl group, and
n indicates a number of X and is an integer of 0 to 4, and when n is two or more, X may be identical to or different from each other),
R¹-Z^{a} (8)
(in formula (8), R¹ is a substituted or unsubstituted C1-6 alkyl group, and Z^{a} is a halogeno group or a group of a formula: R¹O-SO₃-), 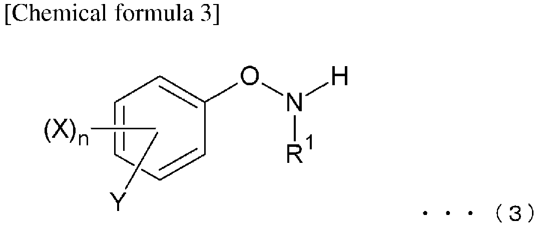 (in formula (3),
R¹ is a substituted or unsubstituted C1-6 alkyl group, and
Y, X and n are defined in formula (2)), 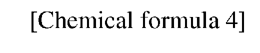 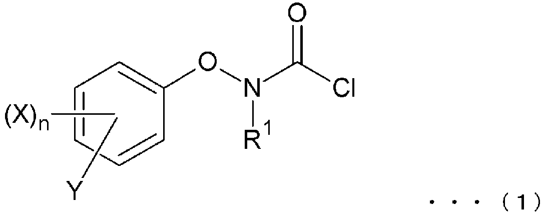 (in formula (1),
R¹, Y, X and n are defined in formula (3)).

3. The production method according to claim 2, further comprising chemically reacting a compound of formula (4) with a compound of formula (5) in the presence of the base to obtain the compound of formula (2), 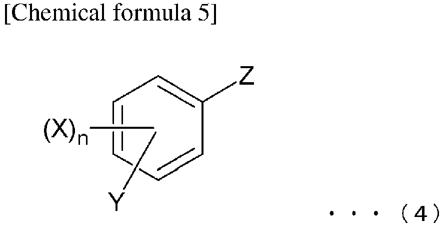 (in formula (4),
Z is a halogeno group, and
X, Y, and n are defined in formula (2)), 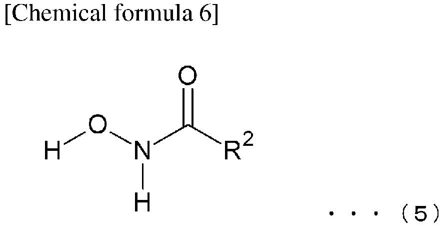 (in formula (5),
R² is defined in formula (2)).

4. The production method according to claim 3, wherein Z is a bromo group.

5. A compound of formula (3): 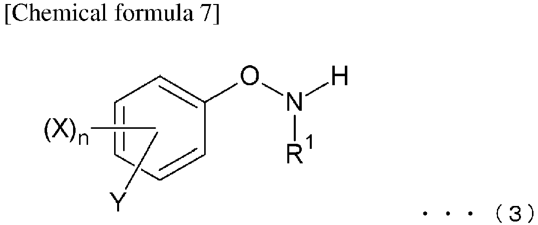 (in formula (3),
R¹ is a substituted or unsubstituted C1-6 alkyl group,
Y is a C1-6 haloalkyl group,
X is a halogeno group, a C1-6 alkyl group, or a C1-6 haloalkyl group, and
n indicates the number of X and is an integer of 0 to 4, and when n is two or more, X may be identical to or different from each other.)

6. The compound according to claim 5, wherein R¹ is a substituted or unsubstituted C2-6 alkyl group.

7. A production method of a compound of formula (3), comprising:
chemically reacting a compound of formula (8) with a compound of formula (2) in the presence of a base to obtain an N-alkylated compound; and
hydrolyzing the N-alkylated compound, 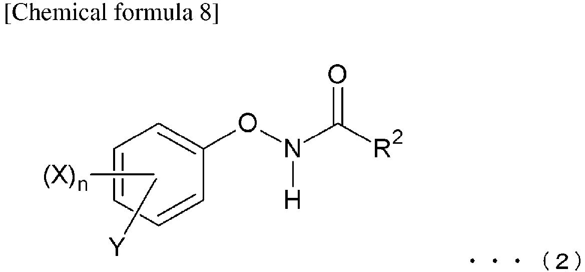 (in formula (2), R² is a C1-8 alkoxy group,
Y is a C1-6 haloalkyl group,
X is a halogeno group, a C1-6 alkyl group, or a C1-6 haloalkyl group, and
n indicates the number of X and is an integer of 0 to 4, and when n is two or more, X may be identical to or different from each other),
R¹-Z^{a} (8)
(in formula (8), R¹ is a substituted or unsubstituted C1-6 alkyl group, and Z^{a} is a halogeno group or a group of formula: R¹O-SO₃-) 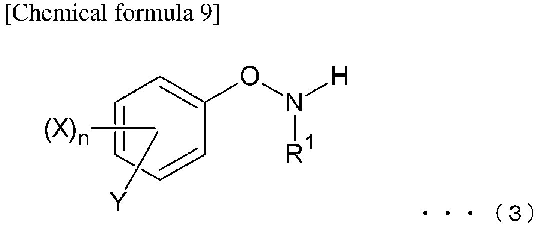 (in formula (3),
R¹ is a substituted or unsubstituted C1-6 alkyl group, and
Y, X and n are defined in formula (2)).

8. The production method according to claim 7, wherein R¹ is a substituted or unsubstituted C2-6 alkyl group.

9. A production method of a compound of formula (1), comprising
chemically reacting a compound of formula (3) with a phosgene or a phosgene equivalent, 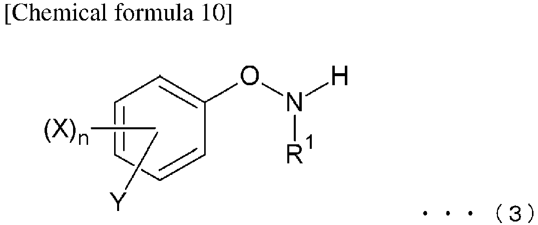 (in formula (3),
R¹ is a substituted or unsubstituted C1-6 alkyl group,
Y is a C1-6 haloalkyl group,
X is a halogeno group, a C1-6 alkyl group, or a C1-6 haloalkyl group, and
n indicates the number of X and is an integer of 0 to 4, and when n is two or more, X may be identical to or different from each other) 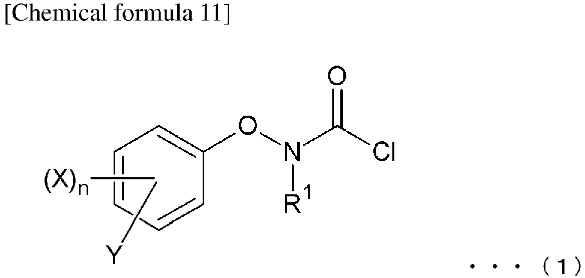 (in formula (1),
R¹, Y, X and n are defined in formula (3)).
